# EUROPEAN PATENT APPLICATION

(11) **EP 2 952 219 A1**
(43) Date of publication of application: **09.12.2015**
(21) Application number: 15170867.4
(22) Date of filing: 05.06.2015
(51) Int. Cl.: A61M 11/00, A61M 11/02, B05B 7/00, A61M 11/06

(54) **DEVICE FOR ATOMIZING ONE OR MORE SUBSTANCES**

(30) Priority: 06.06.2014 IT MI20141046
(71) Applicant: 3A Health Care S.R.L., 25017 Lonato del Garda BS (IT)
(72) Inventor: Abate, Giorgio, I-25017 Lonato del Garda BS (IT)
(74) Representative: Ercolani, Simone Pietro

(57) **Abstract**

Device (1) for atomizing one or more substances, of the type comprising a nebulizer cup (3) for said one or more substances, at least one nozzle (4) arranged at the base (3a) of said nebulizer cup to inject a pressurized gas through at least one outlet port (4a), at least one duct (5) for the air intake into said nebulizer cup and arranged over said at least one nozzle (4), and means (6) to vary the distance between said at least one duct and said one or more outlet ports (4a) of said at least one nozzle (4), said nebulizer cup comprising, on top, at least one passage (7) inside which said at least one duct (5) is, at least in part, movably housed, characterized in that said varying means (6) comprise at least one control knob (8) constrained to said passage (7) so as to at least rotate, in order to at least translationally drive the displacement of said at least one duct (5) along said at least one passage (7).

## Description

The present invention relates to a device for atomizing one or more liquid and/or solid substances, down to particles of the order of few microns. In particular, the present device is used for atomizing drugs, or like products, which have to be inhaled by the patient in order to ensure their therapeutic effectiveness. Such devices are known also as aerosol devices since the final product to be inhaled is a colloid made by a mixture constituted by solid and/or liquid atomized particles of the drug in use and a conveniently selected gas of air type, for example.

It is known that the sizes of the inhaled particles strongly affect the efficiency of the prescribed medical treatment, in fact as smaller is their size as greater their ability of penetrating into the bronchial tree.

In this regard devices are known for atomizing drugs, comprising a nebulizer cup to contain the liquid to be atomized or nebulized, inside which there is a nozzle for injecting a pressurized gas, generally air, and a duct for the fresh air intake from the outside. In practice, the injected pressurized gas is in charge of nebulizing, or atomizing, the substance or the drug present in the nebulizer cup. This is obtained by the pressure drop created at the outlet port of the nozzle, and the consequent entrainment of small amounts of drug which, being hit by the air jet, crush in very tiny drops or particles. Therefore the atomizing ability of an aerosol device, i.e. the ability of obtaining particles having a variable diameter depending on the particular medical treatment prescribed, depends on the characteristics of the nebulizer cup, the pressure of the injected air flow and the flow rate of such a flow.

It is known as well that the atomizing ability of a device also depends on the distance between the lower end of the aforementioned duct for the air intake and the inner nozzle for the injection of a pressurized gas.

In this regard, in fact, devices are known wherein the air intake duct can be replaced by a similar one having however different size, in order to increase or decrease the ability of atomizing the drug or medicament to be inhaled.

Disadvantageously, in such known devices the replacement of the correct air intake duct is rather slow and, however, forces the user to have several inlet ducts available that can be replaced according to the desired nebulization needs, i.e. depending on the type of drug to be inhaled and/or to the region of the bronchial tree to be treated. For example, in EP0653218 an atomizing device is described in which the ending portion of the fresh air intake duct can be replaced by another one of different size such to allow a different atomization of the substance introduced into the container, or nebulizer cup. There is therefore the need of providing several ending portions in order to obtain different atomization effects.

Furthermore, the application ITMI2009U0057 in the owner's name describes an atomizing device comprising means for the relative displacement of the air intake duct with respect to the inlet port of the device nozzle. In particular, such displacing means comprise a rack integral, or integrated, with the outer surface of the air intake duct and a knob integral to the upper portion of the nebulizer cup and provided with a toothing meshing, when the knob is rotated, the rack for the translational displacement of the duct. However, such a solution assumes that the nebulizer cup is provided with an additional hole placed laterally to the nebulizer cup, so that the knob can interact with the duct. Obviously, such a solution increases the structural complexity of the nebulizer cup and, thus, requires longer assembling times.

Object of the present invention is to realize an atomizing device which is structurally easier than those of known art as well as easier to assemble.

Further object of the invention is to realize an atomizing device which is also easier and more user-friendly to use.

These and other objects are reached by the device for atomizing one or more substances, of the type comprising a nebulizer cup for said one or more substances, at least one nozzle arranged at the base of said nebulizer cup to inject a pressurized gas through at least one outlet port, at least one duct for the air intake into said nebulizer cup and arranged over said at least one nozzle, and means to vary the distance between said at least one duct and said one or more outlet ports of said at least one nozzle, said nebulizer cup comprising, on top, at least one passage inside which said at least one duct is, at least in part, movably housed, characterized in that said varying means comprise at least one control knob constrained to said passage so as to at least rotate, in order to at least translationally drive the displacement of said at least one duct along said at least one passage.

In practice, such a solution removes the need of realizing an additional passage, or hole, in the nebulizer cup and of taking advantage from the already existing one at the passage inside which the duct is housed for positioning a control knob that allows the displacement of the duct to different distances from the nozzle. This obviously remarkably simplifies not only the shape of the die of the nebulizer cup, but also the assembling of the final device and makes easier its use by the user.

According to the invention, said duct comprises an upper portion which is at least partially arranged outside of said nebulizer cup, on top of said at least one control knob. In particular, said upper portion of said duct is shaped to interfere with the upper portion of said at least one passage, or said at least one knob, and limit the lower sliding of said duct in said passage. In this way the duct cannot completely be retracted in the nebulizer cup and is however maneuverable from the outside of the nebulizer cup itself.

According to an embodiment of the invention, said upper portion of said at least one duct comprises said at least one control knob. In practice, said control knob constitutes the upper portion of the duct and is further translationally constrained with respect to said passage in order to drive the rotational and translational displacement of said duct in said passage. In practice, the knob can be grabbed by the user from the outside of the nebulizer cup in order to displace the duct to various distances from said one or more outlet ports of said nozzle.

According to a further embodiment, said at least one control knob, which is separated from said upper portion of said duct, is arranged underneath said upper portion of said duct and is provided with a pierced portion in which, at least partially, said at least one duct is housed and inside which said at least one duct is translationally movable at least when said at least one knob is rotated with respect to said at least one passage. In such an embodiment the knob is evidently separated from the duct. In particular, said passage comprises at least one guide for the mere translation of said duct along said passage.

Furthermore, said device comprises means for stably and reversibly engaging said at least one duct to said at least one nebulizer cup, or said at least one knob, at a plurality of different distances with respect to said one or more outlet ports of said at least one nozzle.

In particular, in accordance with the embodiment of the invention wherein the upper portion of the duct comprises said control knob, said engaging means are provided with at least two notches having different depths and obtained in the lower end of said duct, in different angular positions, and with at least one tooth integral with said at least one nebulizer cup, or vice versa; each of said at least two notches is couplable by shape to said at least one tooth at least when said control knob is translated and rotated with respect to said nebulizer cup.

In accordance with the embodiment of the invention wherein said upper portion of said duct is separated from said control knob, said engaging means comprise at least two notches having different depths and obtained on the upper portion of said control knob, in different angular positions, and at least one tooth integral with said upper portion of said duct, or vice versa; each of said at least two notches is couplable by shape to said at least one tooth at least when said control knob is rotated with respect to said duct.

Furthermore, still according to the invention, the device comprises at least one first abutting element integrally constrained to said duct, at least one second abutting element constrained to said nebulizer cup, or to said knob, concentrically to said passage, and at least one spring directly or indirectly arranged between said at least one first and said at least one second abutting elements, in order to restrain said duct at said plurality of different distances.

Always according to the invention, said at least one duct further comprises at least one lower portion integrally constrained to said upper portion, i.e. the duct is made of two different parts. In this way, the duct can be assembled easily and rapidly while enabling the implementation of the embodiment of the device according to the invention.

For illustration purposes only, and without limitation, several particular embodiments of the present invention will be now described referring to the accompanying figures, wherein:
figure 1a is an axonometric view of the atomizing device according to a first embodiment of the invention, in a first position of the duct, i.e. at a first distance of the duct with respect to the nozzle port;
figure 1b is a longitudinal sectional view of the device of figure 1a;
figure 2a is an axonometric view of the atomizing device according to a first embodiment of the invention, in a second position of the duct, i.e. at a second distance of the duct with respect to the nozzle port;
figure 2b is a longitudinal sectional view of the device of figure 2a;
figure 3a is an axonometric view of the atomizing device according to a first embodiment of the invention, in a third position of the duct i.e. at a third distance of the duct with respect to the nozzle port;
figure 3b is a longitudinal sectional view of the device of figure 3a;
figure 4 is an exploded view of the duct and the means for varying the distance between said duct and the nozzle port;
figure 5 is an axonometric view of the upper portion of the nebulizer cup;
figure 6a is an axonometric view of the atomizing device in accordance with a second embodiment of the invention, in a first position of the duct, i.e. at a first distance of the duct with respect to the nozzle port;
figure 6b is a longitudinal sectional view of the device of figure 6a;
figure 7a is an axonometric view of the atomizing device in accordance with a second embodiment of the invention, in a second position of the duct, i.e. at a second distance of the duct with respect to the nozzle port;
figure 7b is a longitudinal sectional view of the device of figure 7a;
figure 8a is an axonometric view of the atomizing device in accordance with a second embodiment of the invention, in a third position of the duct, i.e. at a third distance of the duct with respect to the nozzle port;
figure 8b is a longitudinal sectional view of the device of figure 8a;
figure 9 is an exploded view of the duct and the means for varying the distance between said duct and the nozzle port of said second embodiment.

Referring in particular to such figures the generic atomizing device according to the invention is denoted with numeral 1.

The device 1 for atomizing a substance, for example a drug, comprises a nebulizer cup 3 for containing such a drug, a nozzle 4 arranged at the base 3a of the nebulizer cup 3 for injecting pressurized air through an outlet port 4a, and a duct 5 for air intake into the nebulizer cup 3. Note that the injected gas can also be different from air, as well as the number of outlet ports of the nozzle can also be different from one, for example, two or more, without thereby departing from the protection scope of the present invention. The number of substances inside the container can also be different from one, for example, it can consist of a mixture of drugs both in solid and liquid form without thereby departing from the protection scope of the present invention.

Furthermore, the device 1 comprises, as known, a channel 40 for delivering the aerosol mixture composed of the atomized substance and of said gas injected through the afore mentioned two outlet ports 4a of the nozzle 4, and an element 50 against which the pressurized gas outflowing from the port 4a of the nozzle 4 hits. Furthermore, as visible in the accompanying figures, the duct 5 is substantially concentric to the outlet port 4a of the nozzle 4 and is arranged on the upper part of the nebulizer cup 3 itself, above the nozzle 4. Furthermore, the nebulizer cup 3 comprises an upper portion 3c and a lower portion 3b hermetically coupled to the upper portion 3c.

Again, the device 1 comprises means 6 for varying the distance between the duct 5 and the outlet port 4a of the nozzle 4.

According to the invention, the nebulizer cup 3 comprises, on top, a passage 7 inside which the duct 5 is movably housed, whereas the said varying means 6 comprise a control knob 8 rotatably constrained to the passage 7, coaxially to the duct 5, in order to drive the displacement of the duct 5 along the passage 7. According to the particular embodiment described hereinbelow, the passage 7 is made in a single piece with the upper portion 3c of the nebulizer cup 3.

Still according to the invention, the duct 5 comprises an upper portion 5a which is at least in part arranged outside of the nebulizer cup 3, above the control knob 8. In particular, the upper portion 5a of the duct 5 is shaped to interfere with the control knob 8 and, thus, also indirectly with the upper portion 7a of the passage 7, in order to limit, underneath, the sliding of the duct 5 in the passage 7. In practice, therefore, the duct 5 can not be completely retracted in any way within the inside of the nebulizer cup 3.

In this embodiment, the control knob 8 is arranged underneath the upper portion 5a of the duct 5 and is provided with a pierced portion 8b inside which the duct 5 itself is partially housed and inside which still the duct 5 itself is translationally movable when the knob 8 is rotated with respect to the passage 7, i.e. with respect to the nebulizer cup 3.

According to the embodiment described in the accompanying figures, the duct 5 is only translationally constrained to the passage 7 and the control knob 8, which is separated from the duct 5, is arranged underneath the upper portion 5a of the duct 5, in order to drive the translation of the duct along the passage 7. In practice, upon the rotation of the knob 8 the duct 5 axially moves along the passage 7, thus modifying its own distance from the outlet port 4a of the nozzle 4. In particular, the passage 7 comprises a couple of guides 15 for the translation of the duct 5 along the passage 7. Such guides 15, obtained at the lower portion 7b of the passage 7, couple by shape to corresponding guides 5d of the duct 5.

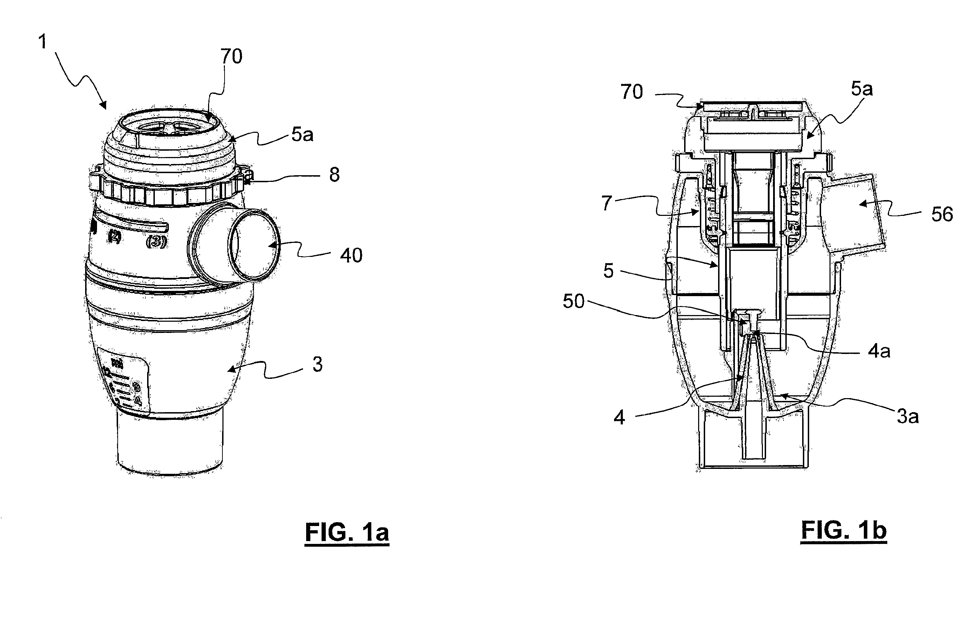

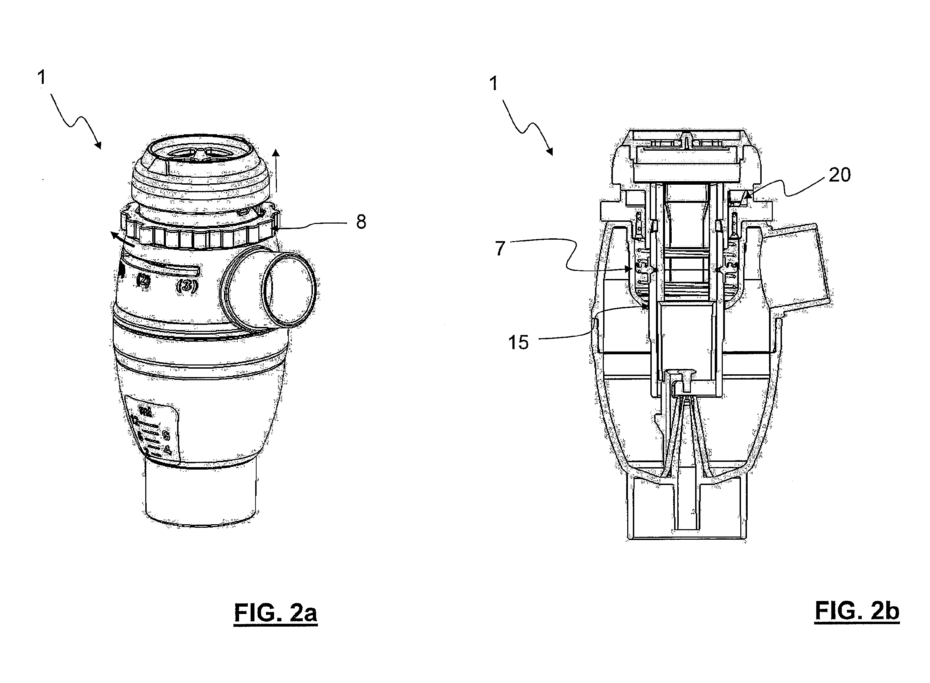

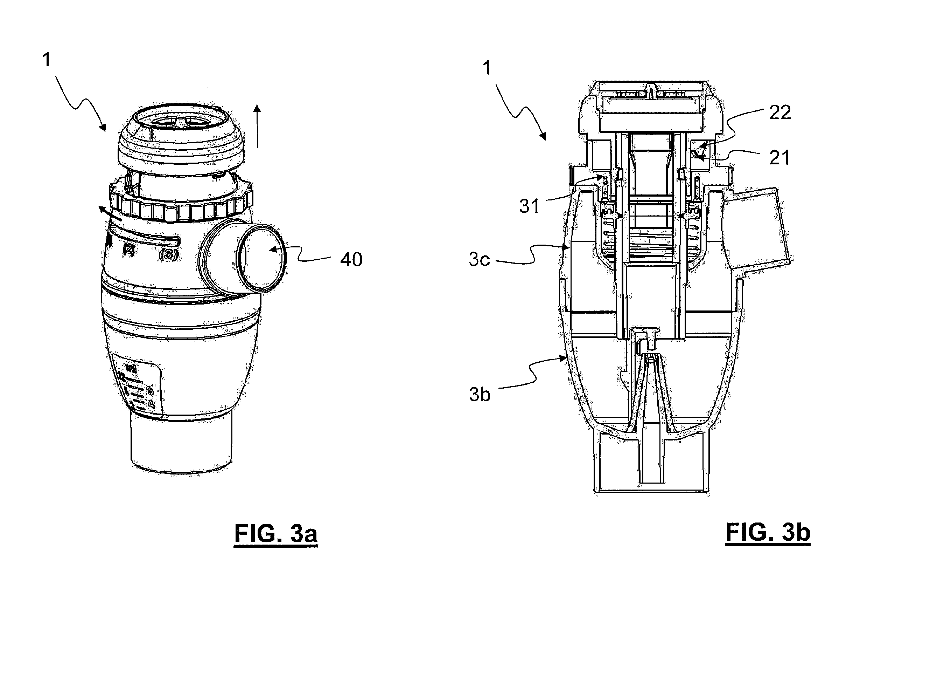

Always according to the invention, the device 1 comprises means 20 for stably and reversibly engaging the duct 5 to the knob 8 at three distinct distances (see figures 1, 2 and 3) with respect to said outlet port 4a of the nozzle 4, that is when the distance between the duct 5 and the outlet port 4a of the nozzle 4 varies.

In detail, such engaging means 20 comprise three notches 21 having different depths and obtained on the upper portion 8a of the control knob 8, in different angular positions, and a tooth 22 integral with the upper portion 5a of the duct 5. Each notch 21 can be coupled by shape to the tooth 22 at least when the control knob 8 is rotated with respect to the duct 5 so that the latter takes three distinct positions at different distances from the outlet port 4a of the nozzle 4. In practice, therefore, when the knob 8 is rotated by the user, the tooth 22 slides between a notch 21 and the other one, getting coupled to one of such notches 21 according to user's needs.

As visible in the accompanying figures, the device 1 further comprises a first abutting element 30 integrally constrained to the duct 5 at the lower portion 5b of the latter, a second abutting element 31 made in a single piece with the knob 8 and concentric to the passage 7, and a compression spring 32 directly arranged between the first abutting element 30 and the second abutting element 31, to stably and reversibly restrain the duct 5 at three different distances with respect to the outlet port 4a of the nozzle 4. In fact the spring 32, already preloaded mounted between the first abutting element 30 and the second abutting element 31, is increasingly compressed switching from a stable position to another one, as the duct 5 takes an increasingly outer position with respect to the nebulizer cup 3 (from figure 1 to figure 3). In fact, the first abutting element 30, in such cases, gets closer to the second abutting element 31. In general, it has to be noted that the first abutting element 30 is shaped so as to interfere with the second abutting element 31, in this case coincident with the control knob 8 in order to limit, on top, the sliding of the duct 5 in the passage 7 in such a way to prevent the duct 5 from exiting outside of the nebulizer cup 3.

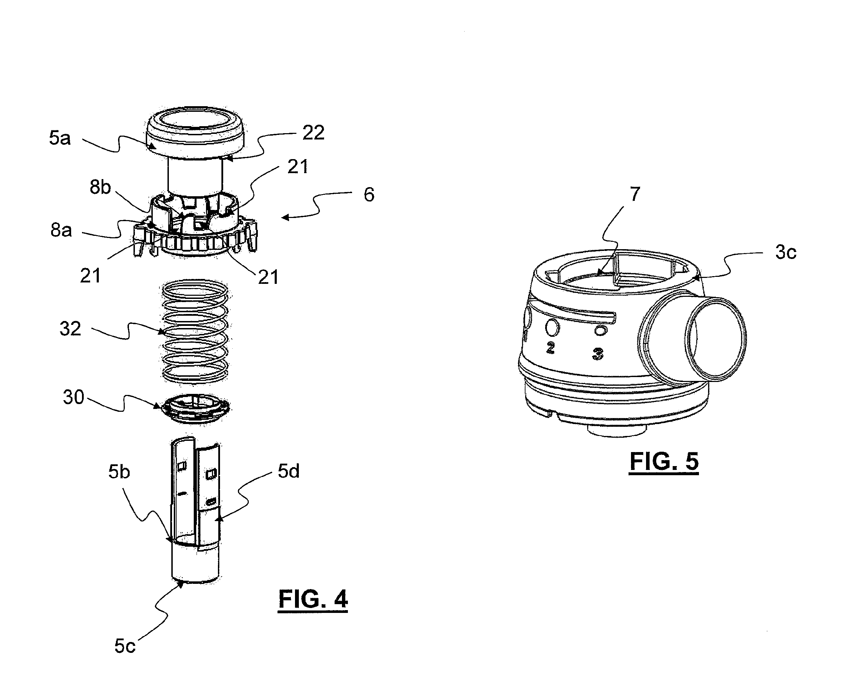

As visible in figure 4, the duct 5 further comprises a lower portion 5b separated from and integrally constrained to the upper portion 5a. In this way, the assembling of the device 1 is made possible since the upper 5a and lower 5b portion of the duct 5 are housed in the passage 7, respectively, from the outside and from the inside of the upper portion 3c of the nebulizer cup 3.

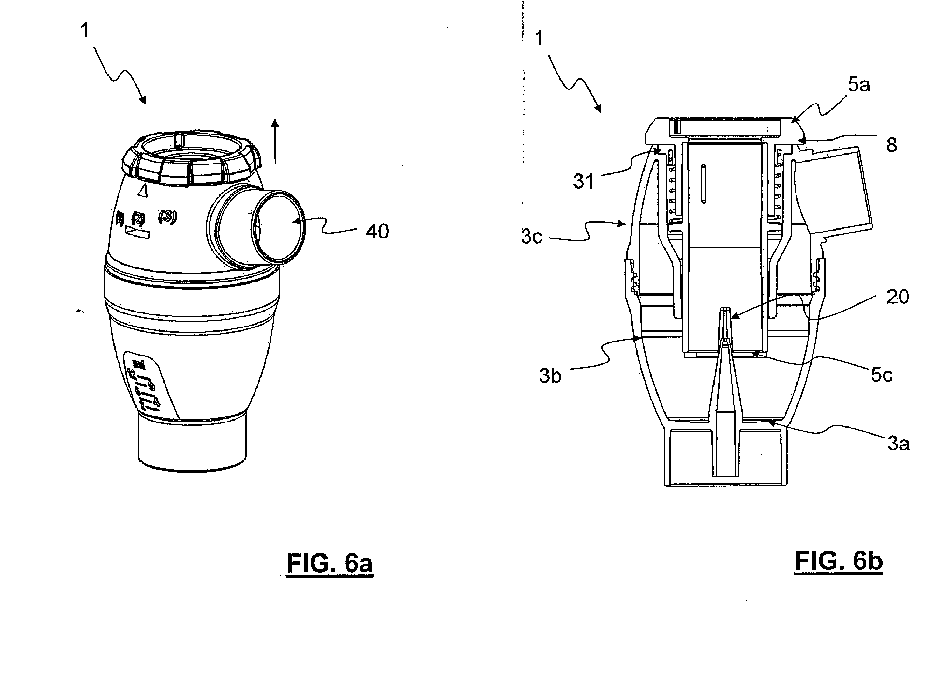

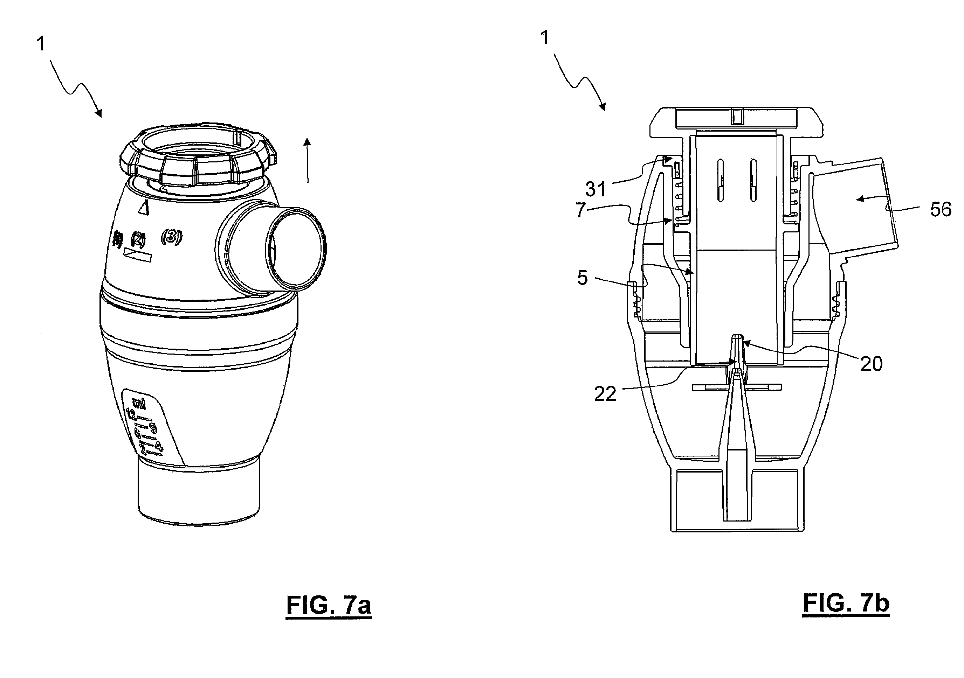

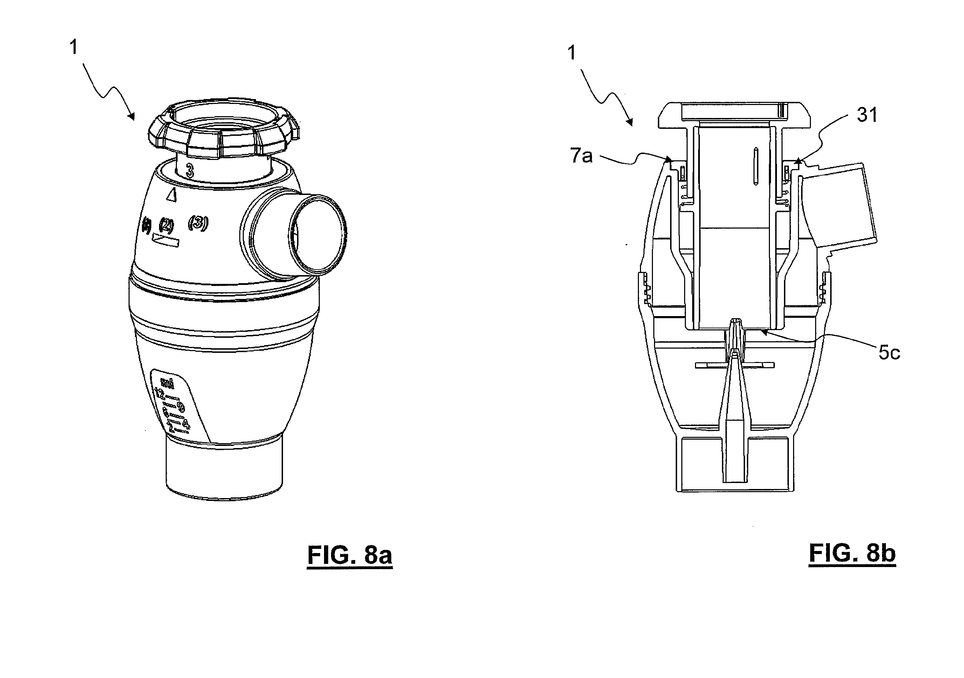

In accordance with a further embodiment depicted in figures 6a, 6b, 7a, 7b, 8a, 8b and 9, the upper portion 5a of the duct 5 comprises the same control knob 8. In practice, the upper portion 5a itself is the control knob 8 for the duct 5. Such an upper portion 5a of the duct 5, practically coinciding with the knob 8, is shaped to interfere with the upper portion 7a of the passage 7 in order to limit, underneath, the sliding of the duct in said passage 7.

Note that, as in the preceding embodiment, the duct 5 further comprises a lower portion 5b separated from and integrally constrained to the upper portion 5a. In this way, the assembling of the device 1 is made possible since the upper 5a and lower 5b portion of the duct 5 are housed in the passage 7, respectively, from the outside and from the inside of the upper portion 3c of the nebulizer cup 3.

Such a control knob 8, on the contrary of that one described in the embodiment depicted in figures 1a to 5, is both rotatably and translationally constrained with respect to the passage 7 of the nebulizer cup 3, in order to thereby drive the rotational and translational displacement of the duct 5 in the passage 7.

Differently from the preceding embodiment the engaging means 20, which are always provided with three notches 21 having different depths, are obtained on the lower ending 5c of the duct 5 in different angular positions, precisely at 120° one from another. Furthermore, the engaging means 20 are provided with a tooth 22 this time integral with the nebulizer cup 3. Each notch 21 is therefore couplable by shape to the tooth 22 at least when said control knob 8 is translated and rotated with respect to the nebulizer cup 3. In practice, in order to vary the distance of the duct 5 with respect to the outlet port 4a, at first the user has to raise and then rotate the knob 8, which specifically also corresponds to the upper portion 5a of the duct 5, so as to move the duct to a position further or closer to the port 4a of the nozzle 4. At this point, as mentioned above, the duct reaches a stable and reversible position at the moment one out of the three notches 21 couples to the tooth 22.

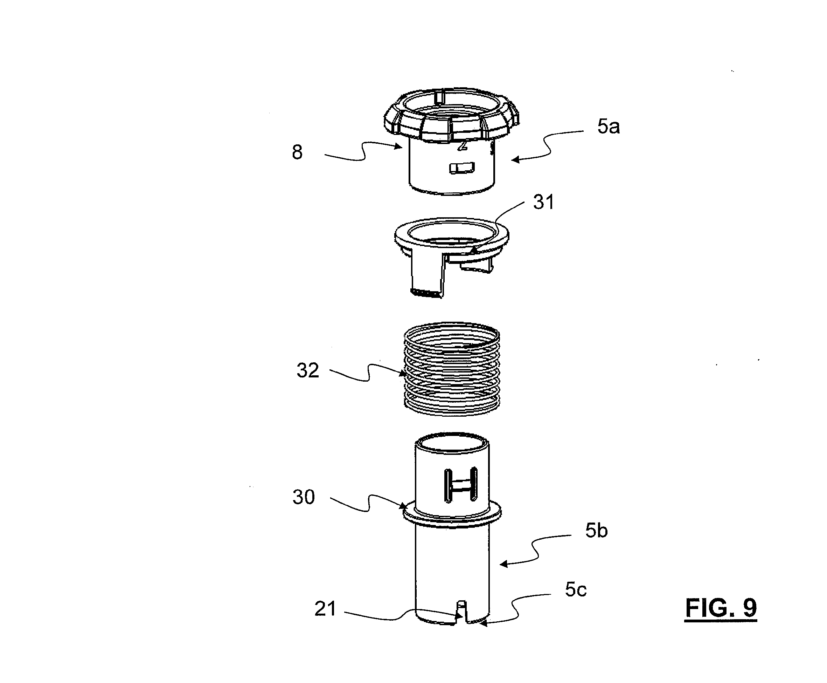

As better visible in figure 9, the device 1 further comprises a first abutting element 30 made in a single piece with the duct 5, at the lower portion 5b of the latter, a second abutting element 31, integrally constrained to the passage 7, at the upper portion 7a, and a compression spring 32 directly arranged between the first abutting element 30 and the second abutting element 31, to stably and reversibly restrain the duct 5 at three different distances with respect to the outlet port 4a of the nozzle 4. In fact the spring 32, already preloaded mounted between the first abutting element 30 and the second abutting element 31, is increasingly compressed switching from a stable position to another one, as the duct 5 takes an increasingly outer position with respect to the nebulizer cup 3 (from figure 6a to figure 8b). In fact, the first abutting element 30, in such cases, gets closer to the second abutting element 31. In general, it has to be noted that the first abutting element 30 is shaped so as to interfere with said second abutting element 31 in order to limit, on top, the sliding of the duct 5 in the passage 7, in such a way to prevent the duct 5 from exiting outside of the nebulizer cup 3.

Finally, it has to be noted that in both the embodiments herein described, but preferably as shown in the first embodiment, above the duct 5, a filter can be arranged for the air entering the duct 5 itself.

## Claims

1. Device (1) for atomizing one or more substances, of the type comprising a nebulizer cup (3) for said one or more substances, at least one nozzle (4) arranged at the base (3a) of said nebulizer cup to inject a pressurized gas through at least one outlet port (4a), at least one duct (5) for the air intake into said nebulizer cup and arranged over said at least one nozzle (4), and means (6) to vary the distance between said at least one duct and said one or more outlet ports (4a) of said at least one nozzle (4), said nebulizer cup comprising, on top, at least one passage (7) inside which said at least one duct (5) is, at least in part, movably housed, **characterized in that** said varying means (6) comprise at least one control knob (8) constrained to said passage (7) so as to at least rotate, in order to at least translationally drive the displacement of said at least one duct (5) along said at least one passage (7).

2. Device according to claim 1, **characterized in that** said at least one duct comprises an upper portion (5a) that is at least in part arranged outside of said nebulizer cup (3).

3. Device according to claim 1 and 2, **characterized in that** said upper portion (5a) of said at least one duct (5) is shaped to interfere with the upper portion (7a) of said at least one passage (7) or of said at least one control knob (8), in order to limit, at the bottom, the sliding of said at least one duct in said passage.

4. Device according to one or more of the preceding claims, **characterized in that** said upper portion (5a) of said at least one duct (5) comprises said at least one control knob (8), said at least one control knob being translationally constrained as well to said at least one passage (7) in order to rotationally and translationally drive the displacement of said at least one duct (5) in said at least one passage (7).

5. Device according to one or more of claims 1 to 3, **characterized in that** said at least one control knob (8) is arranged underneath said upper portion (5a) of said at least one duct (5) and is provided with a pierced portion inside which said at least one duct (5) is housed, at least in part, and inside which said at least one duct is translationally movable at least when said knob is rotated with respect to said passage.

6. Device according to claim 5, **characterized in that** said at least one passage (7) comprises at least one guide (15) for the translation of said at least one duct along said at least one passage.

7. Device according to one or more of claims 2 to 6, **characterized by** comprising means (20) for stably and reversibly engaging said at least one duct (5) to said nebulizer cup (3), or to said at least one knob (8), at a plurality of different distances with respect to said one or more outlet ports of said at least one nozzle.

8. Device according to one or more of claims 2 to 4 and 7, **characterized in that** said engaging means (20) are provided with at least two notches (21) having different depths and obtained on the lower end (5c) of said at least one duct (5), in different angular positions, and with at least one tooth (22) integral with said nebulizer cup, or vice versa, each of said at least two notches being couplable by shape to said at least one tooth at least when said at least one control knob is translated and rotated with respect to said nebulizer cup.

9. Device according to one or more of claims 5 to 7, **characterized in that** said engaging means (20) comprise at least two notches (21) having different depths and obtained on the upper portion (8a) of said at least one control knob (8), in different angular positions, and at least one tooth (22) integral with said upper portion of said at least one duct, or vice versa, each of said at least two notches being couplable by shape to said at least one tooth at least when said at least one control knob (8) is rotated with respect to said at least one duct (5).

10. Device according to one or more of claims 2 to 9, **characterized by** comprising at least one first abutting element (30) integrally constrained to said at least one duct, at least one second abutting element (31) constrained to said nebulizer cup, or to said at least one knob, concentrically to said passage, and at least one spring (32) directly or indirectly arranged between said at least one first abutting element and said at least one second abutting element, in order to restrain said at least one duct (5) in each of said plurality of different distances.

11. Device according to claim 10, **characterized in that** said at least one first abutting element (30) is shaped to interfere with said at least one second abutting element (31) in order to limit, on top, the sliding of said at least one duct in said at least one passage.

12. Device according to one or more of the preceding claims, **characterized in that** said at least one duct (5) further comprises a lower portion (5b) integrally constrained to said upper portion (5a).

13. Device according to one or more of the preceding claims, **characterized in that** said at least one duct (5) is substantially concentric to said at least one nozzle (4).

14. Device according to one or more of the preceding claims, **characterized in that** said nebulizer cup (3) comprises an upper portion (3c) and a lower portion (3b) hermetically coupled to said upper portion (3c), said upper portion comprising said at least one passage (7) for said at least one duct.
